# EUROPEAN PATENT SPECIFICATION

(11) **EP 1 727 555 B1**
(45) Date of publication and mention of the grant of the patent: **29.04.2009**
(21) Application number: 04802152.1
(22) Date of filing: 15.12.2004
(51) Int. Cl.: A61K 36/185, A61P 35/00, A61P 15/12, A61P 19/10, A61P 5/24, A61P 9/10

(54) **PRODUCTION OF HOP EXTRACTS HAVING OESTROGENIC AND ANTIPROLIFERATIVE BIOACTIVITY**
HERSTELLUNG VON HOPFENEXTRAKT MIT ÖSTROGENER UND PROLIFERATIONSHEMMENDER BIOAKTIVITÄT
PRODUCTION DE D'EXTRAITS DE HOUBLON AYANT UNE BIOACTIVITE OESTROGENIQUE ET ANTI-PROLIFERATIVE

(30) Priority: 16.12.2003 EP 03447290
(43) Date of publication of application: 06.12.2006
(73) Proprietor: Metagenics Belgium BVBA, 8400 Oostende (BE)
(72) Inventor: MAES, Francis, B-8420 De Haan (BE); DE KEUKELEIRE, Denis, B-9090 Melle (BE); HEYERICK, Arne, B-9050 Gentbrugge (BE)
(74) Representative: Luys, Marie-José A.H.
(86) International application number: PCT/BE2004/000176
(87) International publication number: WO 2005/058336

(56) References cited:
- WO-A-02/39960
- WO-A-02/085393
- WO-A-03/014287
- US-A1- 2002 110 579
- HAENSEL R ET AL: "DEMETHYLXANTHOHUMOL ISOLATION FROM HOPS AND CYCLIZATION TO FLAVANONES" ARCHIV DER PHARMAZIE (WEINHEIM), vol. 321, no. 1, 1988, pages 37-40, XP009038591 ISSN: 0365-6233 cited in the application
- MILLIGAN S ET AL: "Oestrogenic activity of the hop phyto-oestrogen, 8-prenylnaringenin" REPRODUCTION (CAMBRIDGE), vol. 123, no. 2, February 2002 (2002-02), pages 235-242, XP009038592 ISSN: 1470-1626 cited in the application

## Description

The present invention relates to a method for the production of hop extracts which are enriched in 8-prenylnaringenin with respect to 6-prenylnaringenin according to the preamble of the first claim.

Hops contains three major classes of secondary metabolites namely the hop (bitter) acids, the hop essential oil, and the hop polyphenols. Hop acids and the hop essential oil, and to a certain amount low-molecular-weight polyphenols, are the most important hop constituents for the purpose of beer brewing. Hop extracts for brewing purposes are generally prepared by extracting hops or hop products with liquid or supercritical CO₂, in view of obtaining hop extracts containing the above-mentioned secondary metabolites in the desired proportions.

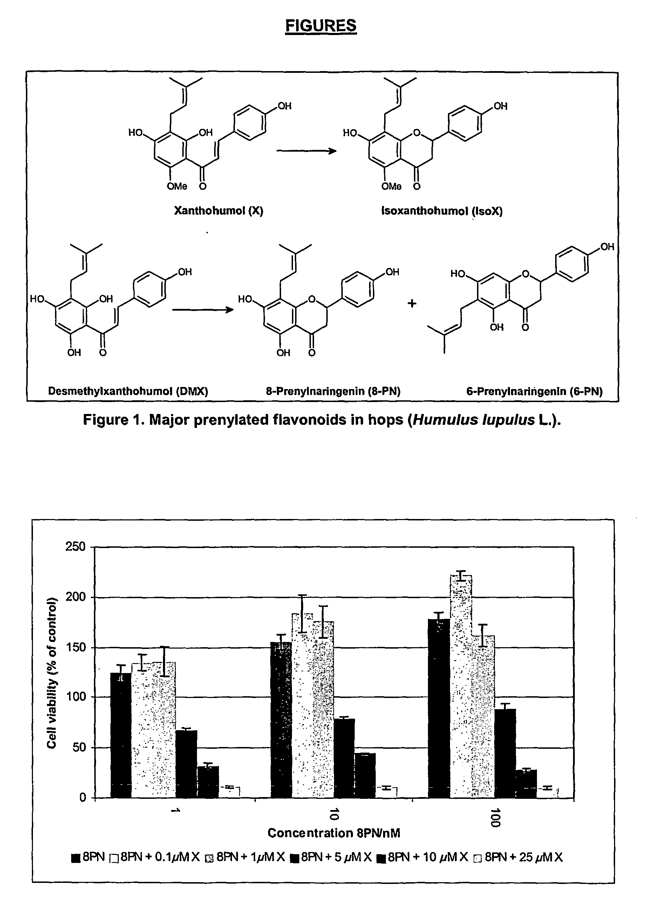

Recently, significant research has been focused on the biological activity of prenylated flavonoids, which constitute a specific class of polyphenols. The most important prenylated flavonoids present in fresh hops are *chalcones,* in particular xanthohumol and desmethylxanthohumol. These chalcones are precursors for *flavanones* such as isoxanthohumol (which originates from xanthohumol), 8-prenylnaringenin and 6-prenylnaringenin (which both originate from desmethylxanthohumol) (see figure 1). 8-Prenylnaringenin has been identified as the active principle of the oestrogenic activity of hops with an activity greater than that of other established phyto-oestrogens (Milligan et al., 1999). Next to 8-prenylnaringenin, other similar hop-derived compounds, such as 6-prenylnaringenin, isoxanthohumol, 6,8-diprenylnaringenin, and 8-geranylnaringenin (Milligan et al., 2000) were found to be only weakly oestrogenic. The high oestrogenic activity of 8-prenylnaringenin was confirmed by *in vivo* tests (Milligan et al., 2002).

Xanthohumol on the other hand has been shown to be a very potent cancer chemopreventive compound *in vitro* with an exceptional broad spectrum of inhibitory mechanisms at the initiation, promotion, and progression stages of carcinogenesis (Gerhauser et al., 2002). Consistent with the anti-initiating potential, xanthohumol potently modulates the activity of enzymes involved in carcinogen metabolism and detoxification, e.g. CYP450-enzymes (Henderson et al., 2000; Miranda et al., 2000c) and quinone reductases (Miranda et al., 2000a). Moreover, xanthohumol has been found capable of scavenging reactive oxygen species including hydroxyl and peroxyl radicals (Miranda et al., 2000b; Rodriguez et al., 2001) and of inhibiting superoxide anion radical and nitric oxide production (Zhao et al., 2003). As potential anti-tumour promoting activity, xanthohumol demonstrates anti-inflammatory properties by inhibition of cyclooxygenase-1 and cyclooxygenase-2 activity (Gerhauser et al., 2002). Antiproliferative mechanisms to prevent carcinogenesis in the progression phase include inhibition of DNA synthesis and induction of cell cycle arrest in the S-phase, apoptosis, and induction of cell differentiation (Miranda et al., 1999; Gerhauser et al., 2002). Furthermore, xanthohumol proved efficient at nanomolar concentrations in preventing carcinogen-induced preneoplastic lesions in mouse mammary gland organ culture, a model that serves as a link between short-term *in vitro* and long-term *in vivo* carcinogenesis models (Gerhauser et al., 2002).

The above-described flavonoids are non-essential products in the process of beer brewing. They largely remain behind in the residue left after extraction of the desired compounds from hops with liquid or supercritical CO₂. In normal brewing conditions, whereby either whole hops, hop products, or particular hop fractions are used, the total concentration of prenylated flavonoids may be up to 4 mg per litre of beer (Stevens et al., 1999).

In DE 199 39 350, a method for the production of a hop extract enriched in xanthohumol is described, according to which a hop product is extracted with an organic solvent or alkaline water. Prior to this extraction, the hop product may be extracted with water to remove hydrophilic substances. As a suitable organic solvent, a water/ethanol solution is disclosed. Nothing however is mentioned about the presence of prenylated flavanones in the hop extract.

According to WO03014287, the oestrogenic activity of hop extracts must primarily be attributed to the presence of 8-prenylnaringenin, which has been found to be the compound showing the most important oestrogenic activity. It is reported that 6-prenylnaringenin also shows oestrogenic activity, although lower than 8-prenylnaringenin and that xanthohumol is capable of interfering in cell metabolism and can be regarded as a cancer-preventing agent. The hop extracts of WO03014287 are disclosed to be suitable for use in the prophylaxis or therapy of disease states caused by a lack of oestrogens or disturbances in the metabolism of sex steroidal hormones, in particular of oestrogens.

WO03014287 also discloses a method for producing a hop extract, which is enriched in chalcones and flavanones, such as xanthohumol, isoxanthohumol, 6-prenylnaringenin and 8-prenylnaringenin. The method comprises the steps of
(1) extracting hops or a hop product with a C₅-C₇ alkane or supercritical CO₂ for removing hydrophobic substances contained in hops,
(2) extracting the residue obtained in step (1) with water in order to remove hydrophilic substances contained in hops and finally
(3) extracting the residue obtained in step (2) with an organic solvent selected from the group consisting of alcohols, water-based alcohols, ketones, water-based ketones or esters or mixtures thereof.
The concentrations of isoxanthohumol, 6-prenylnaringenin and 8-prenylnaringenin in the thus obtained hop extract have been found to increase with an increasing temperature during water extraction in step (2). However, in all examples the weight ratio of 8-PN/(8-PN+6PN) is within the range of 18%-25%.

The hop extract disclosed in WO02/085393 is reported to contain at least 3 wt.% of prenylated flavonoids, in particular xanthohumol, isoxanthohumol, and 8-prenylnaringenin. However, a majority of the extracts of WO02/085393 did not show enhanced oestrogenic activity when compared to a traditional, non-enriched extract. Moreover, no correlation is shown between the composition of the extract and oestrogenic activity. Also, no oestrogenicity is attributed to 8-prenylnaringenin. It is further alleged that the oestrogenic properties of the extract would be particularly expressed when containing 1-30 wt.% of xanthohumol, 0.01-50 wt.% of isoxanthohumol, 0.0005-10 wt.% of 8-prenylnaringenin. The hop extract of WO02/085393 is said to be suitable for manufacturing medicaments having oestrogenic properties, cosmetic compositions, nutritional supplements, and dietary preparations.

Hänsel and Schulz (1988) disclose to subject desmethylxanthohumol and xanthohumol, isolated from freshly harvested hop cones, to isomerisation in a 5% ethanolic potassium hydroxide solution. Xanthohumol was isomerised to isoxanthohumol with a recovery of 80 %, while isomerisation of desmethylxanthohumol gave both 6-prenylnaringenin and 8-prenylnaringenin, with recoveries of 35 % and 30 %, respectively. Thus, an 8-PN/(8-PN+6-PN) ratio of 46% could be obtained, showing that formation of the thermodynamically more stable 6-prenylnaringenin is favoured.

Alternatively, Milligan et al. (2002) disclose to prepare 8-prenylnaringenin via demethylation of isoxanthohumol, which was isolated after isomerisation of the residue of a dichloromethane extract of dried hop cones in a 5% ethanolic potassium hydroxide solution. The demethylation process was carried out using boron trichloride in a mixture of acetonitrile and dichloromethane and was found to have a yield of only 53%.

A number of patents, for example US 5.370.897, US 3.839.588 and US 1.246.425, disclose to subject alpha-acids to an alkaline isomerisation process when producing iso-alpha-acids. However, extracts suitable for such an isomerisation reaction are mostly obtained via extraction with hexane, supercritical or liquid carbon dioxide as extraction solvents. As such extracts do not contain xanthohumol nor desmethylxanthohumol, the isomerized extracts are devoid of the corresponding prenylated flavanones. Furthermore, US 1.274.678 discloses that when the original extract containing alpha-acids is obtained via benzene extraction, it is found essential to remove the prenylated flavonoids, xanthohumol and isoxanthohumol, as they interfere with the production process. Thus, these findings on isomerisation in alkaline medium bear no relevance to the present invention.

Although methods have been developed for enriching hop extracts in prenylated flavonoids, methods to obtain hop extracts in which the highly oestrogenic 8-prenylnaringenin is predominantly present as compared to its isomer, 6-prenylnaringenin, have not been found to date.

It is therefore an object of the present invention to provide a method for the production of hop extract with an improved yield of 8-prenylnaringenin, in particular a method in which 8-prenylnaringenin is predominantly enriched as compared to the isomer 6-prenylnaringenin.

It is a further object of this invention to provide hop extracts with specific and optimised compositions of all relevant prenylated flavonoids or derivatives thereof taking into account their biological activities.

This is achieved with the present invention by means of a process showing the technical features of the characterising part of the first claim.

The method of the present invention is characterised in that hops or a hop product is subjected to (1) an isomerisation reaction in the presence of water as a solvent and in the presence of an amount of a base and (2) at least one extraction.

In the method of the present invention it is preferred to use water as a solvent in the isomerisation reaction.

Hops or a hop product is understood to comprise any form of processed hops, e.g., palletised hops or any hop extract or any hop-derived residue containing prenylated flavonoids.

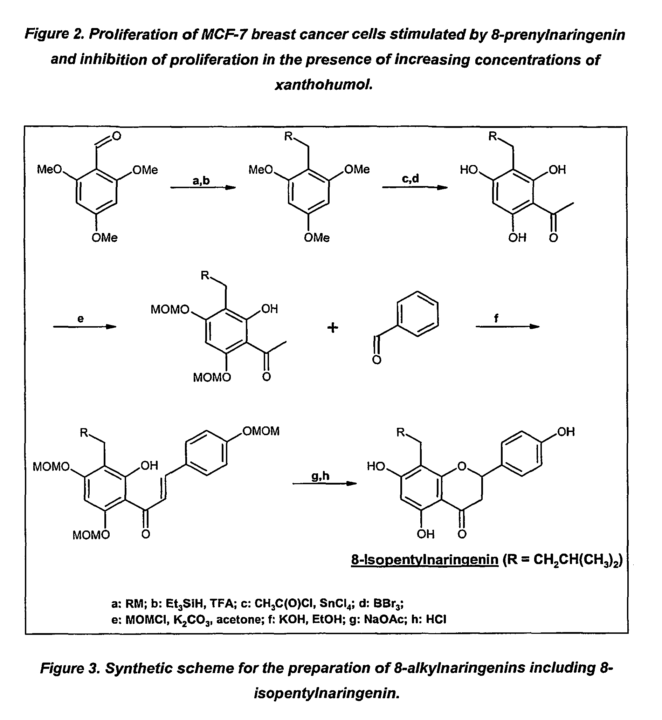

The inventor has now found that when carrying out the isomerisation reaction in the presence of water as a solvent, a hop extract can be obtained which is further enriched in prenylated flavonoids. In particular with the present invention an extract may be obtained which is enriched in 8-prenylnaringenin (and xanthohumol) over 6-prenylnaringenin, as compared to the state of the art, the enrichment being particularly expressed when using exclusively water as a solvent in the isomerisation reaction. Because of the enrichment in 8-prenylnaringenin, a hop extract with a higher oestrogenic activity may be obtained, in which moreover the possible proliferative activity caused by the presence of 8-prenylnaringenin is counteracted by the antiproliferative activity of xanthohumol (see Figure 2). In this respect it is important to note that xanthohumol shows an exceptionally broad spectrum of inhibition mechanisms at all stages of carcinogenesis, i.e., initiation, promotion, and progression (Gerhauser et al., 2002).

The sequence, in which the isomerisation and the at least one extraction are carried out, is not critical to the invention. In practice, this means that either one of the isomerisation or the at least one extraction may be carried out first.

### The isomerisation reaction.

It is known that, at elevated temperatures, both desmethylxanthohumol and xanthohumol - the most important prenylated flavonoids (chalcones) present in hops or a hop product - undergo facile thermal isomerisation through Michael-type intramolecular cycloaddition into their corresponding flavanones. While xanthohumol is virtually exclusively isomerised to isoxanthohumol, isomerisation of desmethylxanthohumol leads to formation of a mixture of 8-prenylnaringenin (8-PN) and 6-prenylnaringenin (6-PN), in a molar ratio of (8-PN x 100%)/(8-PN + 6-PN), which usually is between 15% and 25%, most probably due to the lower thermodynamic stability of 8-PN as compared to 6-PN. It has now surprisingly been found that when carrying out the isomerisation reaction in the presence of water and in specific alkaline conditions, the ratio (8-PN x 100%)/(8-PN + 6-PN) is shifted in favour of the thermodynamically less stable 8-PN, the total yield of (8-PN + 6-PN) remaining almost unaffected. As 8-PN is the compound having the higher oestrogenic activity when compared to 6-prenylnaringenin or any other hop-derived compound, the oestrogenic activity of the thus obtained hop extract may be increased 3-5 times as compared to the known hop extracts (e.g., hydroalcoholic extraction at elevated temperatures). An improved enrichment in 8-PN has been found when using exclusively water as a solvent in the isomerisation reaction.

A preferred embodiment of the method of this invention is characterised in that the isomerisation reaction is carried out in alkaline conditions corresponding to concentrations of KOH in water (w/v%) of at least 0.5, preferably of at least 1, more preferably of at least 5.

Carrying out the isomerisation reaction in increasing alkaline conditions allows to shift the ratio (8-PN x 100%)/(8-PN + 6-PN) from 15-25%, as known from the state of the art, to over 50 % often over 60 and 75 % when using water as a solvent or even higher in favour of 8-PN, while the total yield of (8-PN + 6-PN) remains virtually unaffected. As a result a hop extract may be obtained which is highly enriched in 8-PN. Or in other words, with the present invention the ratio 6-PN/8-PN may be reversed and whereas with the state-of-the-art techniques a selective enrichment in 6-PN was obtained, the present invention provides a selective enrichment in 8-PN, which is the compound having the higher oestrogenic activity. In the reaction conditions prevailing in the method of the present invention, up to approximately 95% of the xanthohumol present in the hop product which is caused to react, may be isomerized to isoxanthohumol. The isomerisation process has been found to proceed in this way also with extracts, in which all desmethylxanthohumol had previously been quantitatively converted to a certain ratio of (8-PN x 100%)/(8-PN + 6-PN).

It is remarked that in the known art, isomerisation of chalcones into their corresponding flavanones is usually carried out in a 5% ethanolic potassium hydroxide (e.g., Hänsel and Schulz, 1988) under reflux conditions for 30 minutes, giving, a (8-PN x 100%)/(8-PN + 6-PN) ratio of 40-46%. With the present invention however, the enrichment of the thermodynamically less stable 8-PN could be significantly increased, by carrying out the isomerisation reaction in water, in particular at ambient temperature.

To minimise the risk to oxidation of the reactants or reaction products, the isomerisation reaction is preferably carried out in inert atmosphere. The reactants, in particular xanthohumol and desmethylxanthohumol as well as the reaction products isoxanthohumol and 8-prenylnaringenin, contain double bonds and phenolic groups, which are susceptible to oxidation.

The temperature at which the isomerisation reaction is carried out is not critical to the invention and may be any temperature between the freeze point and the boiling point of the reaction mixture. Most suitably the reaction is carried out at ambient temperature, although temperatures between 40 and 60°C, in particular about 50°C are also considered suitable.

The inventors have further found that the isomerisation reaction proceeds at a high reaction rate and may virtually be complete within less than 15 minutes, even at ambient temperature. It is preferred to carry out the isomerisation reaction for a time period between 0.25 and 4 h in view of favouring the production of 8-prenylnaringenin. The person skilled in the art will in general be capable of adjusting the duration and the temperature at which the isomerisation reaction is carried out, to obtain optimum conversion and selectivity towards the desired end products and to minimise the risk to the formation of unwanted side products which might occur in case the isomerisation reaction is continued for a too long period of time.

### The extraction process.

To isolate the desired compounds from the hops or hops product either before or after having terminated the isomerisation reaction, the hops or hop product is subjected to at least one extraction.

Extraction of the prenylated flavonoids is obtained by subjecting the hops or hop product to an extraction with at least one organic solvent chosen from the group of alcohols, water-based alcohols, ketones, water-based ketones or esters or mixtures thereof or alkaline water. Examples of organic solvents suitable for use are solvents of medium polarity, e.g., ethyl acetate or acetone, or high polarity, e.g., ethanol or methanol, or mixtures thereof such as a mixture consisting of 90/10 (v/v) ethyl acetate/methanol or a hydroalcoholic mixture (75/25 (v/v) ethanol/water).

Preferably, before or after subjecting the hops or the hop product to the at least one extraction or before or after subjecting the hops or the hop product to the isomerisation reaction, the hops or the hop product is subjected to an additional extraction step with water and/or at least one non-polar organic solvent, followed by recovering the residue or the extract enriched in prenylated flavonoids. The additional extraction with water is done with the purpose of obtaining a higher enrichment of prenylated flavonoids in the enriched hop extract by removing as much as possible any non-functional hydrophilic ballast material present in the enriched extract. Examples of such non-functional ballast material include proteins. An additional extraction with at least one non-polar organic solvent allows to decrease the content of non-functional hydrophobic (lipophilic) ballast material, thus leading to a further higher enrichment or concentration of prenylated flavonoids in the extract enriched in prenylated flavonoids.

In case the concentration of hydrophobic ballast material in the hops or hop product starting material which is to be subjected to the isomerisation reaction and extraction, is too high an additional extraction with at least one non-polar organic solvent may be envisaged. The product subjected to such an additional extraction will mostly be the hop residue remaining after the primary extraction of hops with liquid or supercritical CO₂ performed in non-exhaustive conditions (mild temperature and pressure) to separate the compounds of interest for beer brewing from the residue containing more polar secondary metabolites including relevant prenylated flavonoids.

The extraction of hydrophilic and hydrophobic ballast material can be carried out by any suitable extraction process known to the person skilled in the art. Suitable extraction procedures include solvent-solvent extractions (e.g. water vs. ethyl acetate, hexane vs. hydroalcoholic mixtures) or solid-phase extractions (solubilisation of hydrophilic and hydrophobic material in water and a non-polar organic solvent, respectively, or use of silica or derivatised silica). Separation of the fraction with prenylated flavonoids after extractions with water and the non-polar organic solvent, respectively, can, e.g., be carried out by means of decantation, filtration, and/or centrifugation.

A possible extraction method, for example, involves extracting hops with liquid or supercritical CO₂ and recovering the obtained residue, followed by subjecting the residue to the isomerisation reaction in alkaline conditions. Subsequently, the reaction mixture containing the boiled spent hops is acidified until neutral pH and the residue is recovered, e.g., by filtration or centrifugation after washing with water. The prenylated flavonoids can then be extracted selectively from the residue by using suitable solvents or mixtures thereof.

### Pre-processing

In view of improving the economical feasibility of the present invention and further concentrating the desired compounds, before subjecting it to the combined isomerisation reaction and extraction, the hops or hop product is subjected to an extraction in liquid or supercritical CO₂ or at least one substantially non-polar organic solvent, followed by recovering the residue containing the prenylated flavonoids.

This means in fact that the extract of interest for brewing purposes is recovered, while the residue is enriched in prenylated flavonoids and deprived of a major part of hydrophobic ballast material through pre-extraction. This pre-enriched residue is then subjected to the isomerisation and the at least one extraction in arbitrary order.

Another possibility includes use of liquid or supercritical CO2 extraction of an ethanolic so-called 'pure resin extract', which is mixed with a carrier (e.g., Kieselguhr), and recovery of the residue on the carrier. This residue containing the prenylated flavonoids can then be subjected to the isomerisation and the at least one extraction in any order.

### Appropriate formulation

The inventors have found that a possible proliferation of the enriched hop extract of this invention which is induced by the oestrogenic activity of 8-prenylnaringenin can be inhibited or even counteracted by having an mount of xanthohumol present in the enriched extract, since xanthohumol has been found to show antiproliferative activity. However, the inventors have found that such inhibition can only be obtained under the condition that the weight ratio of xanthohumol to 8-prenylnaringenin is sufficiently high. In particular the inventors have found sufficient inhibition with a xanthohumol to 8-prenylnaringenin weight ration which is at least 10, preferably at least 30. This ration may be obtained by adding xanthohumol in excess to the enriched extract (see figure 2). In other words, at particular weight ratios of xanthohumol to 8-prenylnaringenin, an efficient inhibition of the proliferative activity associated with the oestrogenicity of 8-prenylnaringenin can be obtained by the antiproliferative activity of xanthohumol.

In this respect, figure 2 shows that increasing concentrations of 8-prenylnaringenin lead to increased proliferation of MCF-7 breast cancer cells. The inventors have surprisingly found that for each concentration of 8-prenylnaringenin, low concentrations of xanthohumol (< 1 µM) stimulated the proliferation induced by 8-prenylnaringenin, in particular those concentrations where the ration of xanthohumol with respect to 8-prenylnaringenins is below 10, whereas higher concentrations of xanthohumol of at least 5 µM were found to significantly inhibit the proliferation induced by 8-penylnaringenin.

Unfortunately in the reaction conditions which give the most efficient isomerisation of desmethylxanthohumol to 8-prenylnaringenin and 6-prenylnaringenin and simultaneously favour formation of 8-prenylnaringenin, xanthohumol unfortunately is almost quantitatively isomerised to isoxanthohumol. On the other hand a hop extract, which is enriched in xanthohumol can be produced by subjecting hops or a hop product to at least one extraction under non-isomerising conditions (extraction under neutral or acidic conditions at room temperature). Therefore, preferably the method of the present invention comprises the step of mixing the extract obtained by the above-described method of a combined isomerisation-extraction procedure with a second hop extract enriched in xanthohumol. In that way a hop extract may be obtained containing both xanthohumol and 8-prenylnaringenin, in a specific weight ratio. It is preferred that the weight ratio of xanthohumol to 8-prenylnaringenin is at least 10, more preferably at least 30, as within these ranges a mixture may be obtained showing an appropriate combination of oestrogenic activity, due to the presence of 8-prenylnaringenin, and cancer-chemopreventive activity, due to the presence of xanthohumol. This mixing thus allows compensating for any decrease in the xanthohumol content of the starting product following the at least one isomerisation reaction, due to isomerisation of xanthohumol to isoxanthohumol. By mixing an extract enriched in 8-prenylnaringenin obtained from the combined extraction-isomerisation of this invention with an extract enriched in xanthohumol, the present invention allows to obtain a hop extract with a specific weight ratio of 8-prenylnaringenin to xanthohumol, independent of the degree of conversion of xanthohumol to isoxanthohumol in the isomerisation reaction.

Thus, with the present invention a hop extract can be obtained which contains at least 0.15 wt.% 8-prenylnaringenin and at least 3 wt.% xanthohumol, more preferably of at least 0.33 wt.% 8-prenylnaringenin and at least 10 wt.% xanthohumol.

A suitable method for obtaining the extract enriched in xanthohumol comprises the steps of subjecting hops or a hop product to at least one extraction by means of liquid or supercritical CO₂ or at least one substantially non-polar organic solvent, followed by recovery of the residue enriched in xanthohumol, and subjecting the residue to at least one further extraction with suitable solvents or mixtures thereof in view of further concentrating xanthohumol.

Alternatively, liquid or supercritical CO2 extraction of an ethanolic so-called 'pure resin extract', which is mixed with a carrier (e.g., Kieselguhr) leads to a residue on the carrier enriched in prenylated flavonoids. This residue could then be subjected to at least one extraction with suitable solvents or mixtures thereof.

### Increasing the oestrogenic potential

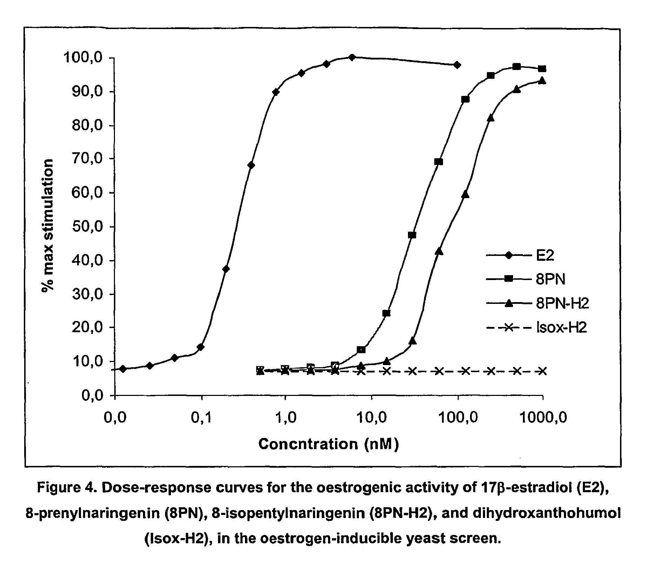

In a further preferred embodiment of the method for the production of a hop extract of the present invention, the method additionally comprises the steps of mixing the extract obtained by the at least one extraction and the isomerisation reaction with an amount of an extract enriched in a compound having high oestrogenic activity such as a 8-alkylnaringening, preferably 8-isopentylnaringenin, which is hydrogenated 8-prenylnaringenin. The reason for the adding 8-isopentylnaringenin is that the inventors surprisingly found that 8-isopentylnaringenin shows oestrogenic activity and that this oestrogenic activity is only 3-4 times weaker than the oestrogenic activity of 8-prenylnaringenin (see figure 4). With respect to 8-isopentylnaringenin it is remarked that although its oestrogenic activity was found to be 3-4 times weaker than that of 8-prenylnaringenin, 8-isopentylnaringenin was found to show a higher bio availability and better stability as compared to 8-prenylnaringenin, as it is less easy metabolised. Most probably this may be attributed to the absence of the double bond in the side chain.

An extract enriched in 8-isopentylnaringenin can be obtained using several methods.

A first possible method includes the steps of:
a. Subjecting a hop extract enriched in xanthohumol to an isomerisation reaction to convert xanthohumol to isoxanthohumol;
b. Subjecting the extract obtained in step (a) to a catalytic hydrogenation reaction to convert isoxanthohumol to dihydro-isoxanthohumol. The catalytic hydrogenation is preferably carried out by admitting H₂ in the presence of a hydrogenation catalyst such as supported Pt or Pd, or any other catalyst. An example of a suitable carrier is activated carbon. The hydrogenation reaction is preferably carried out in MeOH;
c. Subjecting the reaction product of step (b) to a de-methylation reaction. This is done to convert dihydro isoxanthohumol to 8-isopentylnaringenin. The de-methylation reaction may for example be carried out using boron tribromide or any other de-methylating agent.

Thus, with the above described method the oestrogenically inactive xanthohumol may be converted into a compound having high oestrogenic activity, namely 8-isopentylnaringenin. This is a serious advantage as the concentration of xanthohumol in hops is significantly higher (0.3-1.2 wt.%) than the concentration of desmethylxanthohumol (0.005-0.2 wt.%), the precursor of 8-prenylnaringenin. With the above described method a hop extract may be obtained, which is not only enriched in 8-PN but also in 8-isopentylnaringenin, as a result of which the oestrogenic activity may be significantly increased.

A second possible method of obtaining an extract containing 8-isopentylnaringenin is the addition of this product obtained through a synthetic way. Although substitution on the 8-position usually proceeds with low selectivity and yield because a large number of reaction steps are involved, the inventors have now developed a method for the synthesis of 8-isopentylnaringenin, which comprises only a limited number of reaction steps at high yield.

According to this method, 2,4,6-trimethoxybenzaldehyde (see figure 3) is subjected to an alkylation reaction in the presence of an organometallic reagent (e.g., isopentyl lithium), followed by deoxygenation to 1-alkyl-2, 4,6-trimethoxybenzenes (e.g. 1-isopentyl-2, 4,6-trimethoxybenzene). The deoxygenation is preferably carried out in the presence of triethylsilane in trifluoroacetic acid. 1-Alkyl-2, 4,6-trimethoxybenzene is subjected to an acetylation reaction followed by a de-methylation to furnish 3-alkyl-2, 4,6-trihydroxyacetophenones (e.g., 3-isopentyl-2, 4,6-trihydroxyacetophenone). The acetylation reaction is preferably carried out in the presence of acetyl chloride and tin tetrachloride. De-methylation is preferably carried out in the presence of boron tribromide.

In the course of this synthesis route it is preferred to selectively protect two phenolic groups as methoxymethyl ethers (MOM). The third phenol group has been found to resist reaction because of strong intra-molecular hydrogen binding.

The thus obtained de-methylated reaction product is subjected to a mixed aldol reaction involving MOM-protected benzaldehyde, to give formation of chalcones, which undergo an intramolecular Michael-type cycloaddition to the corresponding flavanones. By removing the protective groups in the acidic environment 8-alkylnaringenins (e.g., 8-isopentylnaringenin) are obtained.

The present invention also relates to a hop extract as such, comprising a mixture of 8-prenylnaringenin and 6-prenylnaringenin, wherein the ratio of (8-prenylnaringenin x 100%)/(8-prenylnaringenin + 6-prenylnaringenin) is at least 50%, preferably at least 60%, more preferably at least 75%. Thereby the hop extract preferably comprises an amount of xanthohumol, which is such that the weight ratio of xanthohumol to 8-prenylnaringenin is at least 10, preferably at least 20, more preferably at least 30. It is preferred that the hop extract comprises at least 0.15 % (w/w), preferably at least 0.33% of 8-prenylnaringenin and at least 3 %, preferably at least 10% (w/w) xanthohumol. Most preferably the hop extract further comprises an amount of isoxanthohumol as well as an amount of 8-alkylnaringenin, preferably 8- isopentylnaringenin.

### Indications

The hop extract of the present invention or the hop extract obtainable with the methods of the present invention can be used for the manufacture of a medicament or a phytopharmaceutical having combined oestrogenic and cancer-chemopreventive activities, in which the possible proliferative activity due to the presence of the phyto-oestrogen 8-prenylnaringenin is counteracted by the antiproliferative activity of xanthohumol. Further, the hop extract of the present invention or the hop extract obtainable with the method of the present invention can be used for a medicament or a phytopharmaceutical for the treatment or prophylaxis of conditions or symptoms or complaints or disease states caused by a disturbance in hormonal balance of oestrogenic nature.

Preferably, the condition or symptom or complaint or disease state caused by the disturbance in hormonal balance of oestrogenic nature is related to the menopause (including perimenopause). Thereby preferably, the disease state is osteoporosis. In another preferred embodiment, the disease is selected from the group consisting of sex hormone-dependent cancers, cardiovascular diseases, prostate dysfunction, and colon cancer.
The invention also relates to a nutritional composition/supplement comprising the hop extract of the present invention.

The invention additionally relates to a cosmetic composition comprising the hop extract of the present invention.

The hop extracts resulting from the present invention can be incorporated into various formulations including pills, capsules, gelules, solutions and the likes, while the usual excipients may be applied for best usage and bioavailability.

Additional preferred embodiments are claimed in the dependent claims.

### Examples and figures

The invention is further elucidated in the following examples and figures.

### Example 1. Extraction

Spent hops (hop variety Nugget: 202.74 g), i.e. the residue left after extraction of natural hops with fluid or supercritical CO₂, was extracted by maceration under ambient temperature with 1 l of a 90/10 (v/v) solvent mixture of ethyl acetate and methanol in view of selectively extracting xanthumol and desmethylxanthohumol from the spent hops. The extract was filtered off and the extract enriched in xanthohumol and desmethylxanthohumol (720 ml) was recovered. After evaporating the solvent under reduced pressure, the residue was re-dissolved in 100 ml of a hexane/methanol 1/1 (v/v) mixture with the aim of extracting lipophilic ballast material and transferred to a separatory funnel. After addition of 30 ml of acidified water (1 N HCl), the hexane layer containing the lipophilic ballast material was discarded. The remaining methanolic layer was subjected to a reduced pressure to evaporate the solvent. After addition of water (70 ml) and ethyl acetate (100 ml), and phase separation, the organic layer containing the final hop extract was dried.
Yields:
- Final hop extract 4.64 g (2.3%)
- Desmethylxanthohumol 2.41 wt.%
   *(as measured by HPLC)*
- Xanthohumol 33.62 wt.%
   *(as measured by HPLC)*

### Example 2. Isomerisation

0.5 g of the hop extract obtained in example 1 was stirred for 1 h in water (40 ml) containing varying amounts of KOH (0 g, 0.2 g, 0.4 g, and 2 g, respectively). Subsequently, the solution was acidified to pH = 4-5 using 6 N HCl. The extract was recovered by extraction with ethyl acetate and, after removal of the solvent, dried. The results of the quantitative HPLC-analyses are shown in table 1. It should be noted that the extract, resulting from stirring without the addition of a base, contains 8-prenylnaringenin and 6-prenylnaringenin in a ratio very similar to the commercially available hop extracts, whereas addition of increasing amounts of base significantly increased the ratio (8-prenylnaringenin x 100%)/(8-prenylnaringenin + 6-prenylnaringenin) in favour of 8-prenylnaringenin.

**Table 1. Ratio of (8-prenylnaringenin x 100)/(8-prenylnaringenin + 6-prenylnaringenin) under varying alkaline conditions**

| KOH (w/v %) | Ratio (%) |
|---|---|
| 0 | 22.4 |
| 0.5 | 34.7 |
| 1 | 58.6 |
| 5 | 74.8 |

### Example 3.

A hop extract containing 8-prenylnaringenin and 6-prenylnaringenin was subjected to an isomerisation reaction in 5% aqueous potassium hydroxide at room temperature for 30 minutes. The results of the quantitative HPLC-analyses are shown in table 2. As can be seen from table 2, the ratio (8-prenylnaringenin x 100%)/(8-prenylnaringenin + 6-prenylnaringenin) increased from 23% in the original extract to 73% after isomerisation in favour of 8-prenylnaringenin

### Comparative example A.

The isomerisation reaction of example 3 was repeated, except that instead of 5% aqueous potassium hydroxide use was made of 5% ethanolic potassium hydroxide. The results of the quantitative HPLC-analyses, shown in table 2, show that an extract could be obtained with a ratio of (8-prenylnaringenin x 100%)/(8-prenylnaringenin + 6-prenylnaringenin), which increased from 23% in the original extract to only 43% in the isomerized product.

**Table 2. Composition of a hop extract before and after isomerisation in varying solvents**

| **Composition (in w/w%)** | **Extract before** **isomerisation** | **Extract after** **isomerisation in 5%** **KOH in ethanol** | **Extract after** **isomerisation in 5%** **KOH in water** |
|---|---|---|---|
| 8-Prenylnaringenin (8-PN) | 0,17 | 0,31 | 0,52 |
| 6-Prenylnaringenin (6-PN) | 0,58 | 0,41 | 0,19 |
| **Ratio (8-PNx100%)/(8-PN+6-PN)** | **23%** | **43%** | **73%** |
| Isoxanthohumol | 0,60 | 2,37 | 14,29 |
| Xanthohumol | 14,42 | 12,62 | 0,79 |

### Appropriate formulation

Figure 2 shows the growth stimulation of MCF-7 breast cancer cells by 8-prenylnaringenin and inhibition of this proliferation in the presence of increasing concentrations of xanthohumol.

A bioassay was developed to probe proliferation of oestrogen-dependent cancer cells by 8-prenylnaringenin and to determine concentrations of xanthohumol required to inhibit this 8-prenylnaringenin-induced proliferation. Thereto, oestrogen-responsive MCF-7 breast cancer cells were grown in 96-well plates in the presence of a fixed and growth-stimulatory concentration of 8-prenylnaringenin and varying concentrations of xanthohumol. For each concentration of 8-prenylnaringenin (1 nM, 10 nM, and 100 nM), the concentrations of xanthohumol were 0 µM, 0,1 µM, 1 µM, 5 µM, 10 µM and 25 µM, respectively.

### Increasing the oestrogenic potential

Figure 4 shows the dose-response curves for the oestrogenic activity of 17β-estradiol (E2), 8-prenylnaringenin (8PN), 8-isopentylnaringenin (8PN-H2), and the intermediate dihydro isoxanthohumol (IsoX-H2), as measured with an oestrogen-inducible yeast screen (*Saccharmomyces cerevisiae)* expressing the human oestrogen receptor and containing expression plasmids carrying oestrogen-responsive sequences controlling the reporter gene Lac-Z (Milligan et al., 2001). The yeasts were grown in a medium containing increasing concentrations of 17β-estradiol (positive control), 8-prenylnaringenin (8-PN), 8-isopentylnaringenin (8PN-H2) or dihydro-isoxanthohumol (IsoX-H2). Expression of the Lac-Z reporter gene was spectrophotometrically measured and quantified.

## Claims

1. A method for the production of a hop extract which is enriched in 8-prenylnaringenin with respect to 6-prenylnaringenin, **characterised in that** the method comprises the steps of subjecting hops or a hop product to (1) an isomerisation reaction in the presence of water as a solvent and in the presence of an amount of a base and (2) to at least one extraction.

2. The method according to claim 1, **characterised in that** the isomerisation reaction is carried out in water as a solvent.

3. The method according to claim 1 or 2, **characterised in that** the isomerisation reaction is carried out in alkaline conditions corresponding to concentrations of KOH (w/v%) of at least 0.1, preferably at least 0.5, more preferably of at least 1, most preferably of at least 5.

4. The method according to any one of claims 1-3, **characterised in that**, before subjecting hops or a hop product to the at least one extraction and the isomerisation reaction, the hops or the hop product is subjected to an extraction in the presence of liquid or supercritical CO₂ or at least one substantially non-polar organic solvent, followed by recovering the residue containing the extract enriched in prenylated flavonoids.

5. The method according to any one of claims 1-4, **characterised in that** the method further comprises the mixing of an amount of the hop extract obtained by the at least one extraction and the isomerisation reaction with an amount of a hop extract enriched in xanthohumol.

6. The method according to any one of claims 1-5, **characterised in that** the isomerisation reaction and the at least one extraction are continued until an extract is obtained which contains at least 0.15 wt.%, preferably at least 0.33 wt.% of 8-prenylnaringenin and at least 3 wt.%, preferably at least 10 wt.% of xanthohumol.

7. The method according to any one of claims 1-6, **characterised in that** the isomerisation reaction and the at least one extraction are continued until an extract is obtained with a xanhtohumol/8-prenylnaringenin ratio of at least 10, preferably at least 30.

8. The method according to any one of claims 1-7, **characterised in that** the isomerisation reaction and the at least one extraction are continued until an extract is obtained which contains 6-prenylnaringenin and 8-prenylnaringening in a ratio (8-prenylnaringenin x 100%)(8-prenylnaringenin + 6-prenylnaringenin) of at least 50%, preferably at least 60%, more preferably at least 75%.

9. The method according to any one of claims 1-8, **characterised in that**, as a hop product use is made of a hop product that has been subjected to an additional extraction step with water and/or at least one non-polar organic solvent, followed by recovering the residue containing the extract enriched in prenylated flavonoids.

10. The method according to any one of claims 1-9, **characterised in that** the at least one extraction is carried out with at least one organic solvent chosen from the group of alcohols, water-based alcohols, ketones, water-based ketones or esters or mixtures thereof or alkaline water.

11. The method according to any one of claims 1-10. **characterised in that** the isomerisation reaction is carried out at a temperature between the freeze point and boiling temperature of the reaction mixture, preferably at ambient temperature.

12. The method according to claim 11, **characterised in that** the isomerisation reaction is carried out at a temperature between ambient temperature and 60°C, preferably at a temperature about ambient temperature.

13. The method according to any one of claims 1-12, **characterised in that** the isomerisation reaction is carried out in inert atmosphere.

14. The method according to any one of claims 1-13, **characterised in that** the isomerisation reaction is carried out for a time period between 0.25 and 4 h.

15. The method according to any one of claims 1-14, **characterised in that** the method further comprises the step of mixing an amount of the enriched hop extract obtained from the at least one extraction and the isomerisation reaction with an amount of a hop extract enriched in a 8-alkylnaringenin, preferably 8-isopentylnaringenin.

16. The method according to claim 15, **characterised in that** the hop extract enriched in 8-isopentylnaringenin, is obtained with a method comprising the steps of:
(a) subjecting a hop extract enriched in xanthohumol to an isomerisation reaction to convert xanthohumol to isoxanthohumol;
(b) subjecting the extract obtained in step (a) to a catalytic hydrogenation reaction to convert isoxanthohumol to dihydroisoxanthohumol;
(c) subjecting the extract obtained in step (b) to a demethylation reaction to convert dihydroisoxanthohumol to 8-isopentylnaringenin.

17. The method according to claim 16, **characterised in that** the isomerisation reaction in step a) is carried out in alkaline conditions.

18. The method according to claims 15-17, **characterised in that** the hop extract enriched in 8-alkylnaringenin is obtained by addition of an amount of a synthetic 8-alkylnaringenin, preferably an amount of synthetic 8-isopentylnaringenin.

19. A hop extract comprising a mixture of 8-prenylnaringenin and 6-prenylnaringenin, wherein the ratio of (8-prenylnaringenin x 100%)/(8-prenylnaringenin + 6-prenylnaringenin) is at least 50%, preferably at least 60%, more preferably at least 75%.

20. A hop extract as claimed in claim 19, **characterised in that** the extract comprises a mixture of xanthohumol and 8-prenylnaringenin, the weight ratio of xanthohumol to 8-prenylnaringenin being at least 10, preferably at least 20, more preferably at least 30.

21. A hop extract as claimed in claim 19 or 20, **characterised in that** the hop extract comprises at least 0.15 % (w/w), preferably at least 0.33% of 8-prenylnaringenin and at least 3 %, preferably at least 10% (w/w) xanthohumol.

22. A hop extract as claimed in any one of claims 19-21, **characterised in that** the hop extract further comprises isoxanthohumol.

23. A hop extract as claimed in any one of claims 19-22, **characterised in that** the hop extract further comprises an amount of 8-alkylnaringenin, preferably 8- isopentylnaringenin.

24. Use of the hop extract according to any one of claims 19-23 or the hop extract obtainable with the method of any one of claims 1-18 for the manufacture of a medicament or a phytopharmaceutical in which the possible proliferative activity, due to the estrogenic activity of 8-prenylnaringenin is inhibited (or counteracted) by the antiproliferative activity of xanthohumol.

25. Use of the hop extract according to any one of claims 19-23 or the hop extract obtainable with the method of any one of claims 1-18 for the manufacture of a medicament or a phytopharmaceutical product for the treatment or prophylaxis of any one of conditions, symptoms, complaints or disease states or a combination thereof caused by a disturbance in hormonal balance of oestrogenic nature.

26. Use as claimed in claim 25, whereby the condition, symptom, complaint or disease state caused by the disturbance in hormonal balance of oestrogenic nature is the menopause.

27. Use of the hop extract as claimed in claim 25, whereby the disease state is osteoporosis.

28. Use of the hop extract as claimed in claim 25, whereby the disease state is selected from the group consisting of sex hormone-dependent cancers, cardiovascular diseases, prostate dysfunction, colon cancer.

29. A nutritional composition/supplement comprising an amount of the hop extract according to any one of claims 19-23.

30. A cosmetic composition comprising an amount of the hop extract according to any one of claims 19-23.

## Patentansprüche

1. Verfahren zur Herstellung eines Hopfenextraktes, der in Bezug auf 6-Prenylnaringenin mit 8-Prenylnaringenin angereichert ist, **dadurch gekennzeichnet, dass** das Verfahren folgende Schritte umfasst, in denen der Hopfen oder ein Hopfenprodukt (1) einer Isomerisationsreaktion in Gegenwart von Wasser als Lösemittel und in Gegenwart einer Menge einer Base und (2) mindestens einer Extraktion unterzogen wird.

2. Verfahren nach Anspruch 1, **dadurch gekennzeichnet, dass** die Isomerisationsreaktion in Wasser als Lösemittel erfolgt.

3. Verfahren nach Anspruch 1 oder 2, **dadurch gekennzeichnet, dass** die Isomerisationsreaktion unter basischen Bedingungen erfolgt, welche Konzentrationen von KOH (Gew./Vol.%) von mindestens 0,1, vorzugsweise mindestens 0,5, bevorzugter mindestens 1 und am bevorzugtesten mindestens 5 entspricht.

4. Verfahren nach einem der Ansprüche 1-3, **dadurch gekennzeichnet, dass**, bevor der Hopfen oder ein Hopfenprodukt der mindestens einen Extraktion und der Isomerisationsreaktion unterzogen wird, der Hopfen oder das Hopfenprodukt einer Extraktion in Gegenwart von flüssigem oder superkritischem CO₂ oder mindestens einer Behandlung mit einem im Wesentlichen nichtpolaren organischen Lösemittel unterzogen wird, gefolgt von der Wiedergewinnung des Restes, welcher den Extrakt angereichert mit prenylierten Flavonoiden enthält.

5. Verfahren nach einem der Ansprüche 1-4, **dadurch gekennzeichnet, dass** das Verfahren ferner das Mischen einer Menge des Hopfenextraktes erhalten durch die mindestens eine Extraktion und die Isomerisationsreaktion mit einer Menge eines Hopfenextraktes angereichert mit Xanthohumol umfasst.

6. Verfahren nach einem der Ansprüche 1-5, **dadurch gekennzeichnet, dass** die Isomerisationsreaktion und die mindestens eine Extraktion fortgesetzt werden, bis ein Extrakt erhalten wird, welcher mindestens 0,15 Gew.%, bevorzugt mindestens 0,33 Gew.% 8-Prenylnaringenin und mindestens 3 Gew.%, bevorzugt mindestens 10 Gew.% Xanthohumol enthält.

7. Verfahren nach einem der Ansprüche 1-6, **dadurch gekennzeichnet, dass** die Isomerisationsreaktion und die mindestens eine Extraktion fortgesetzt werden, bis ein Extrakt mit einem Xanthohumol/8-Prenylnaringenin-Verhältnis von mindestens 10, vorzugsweise mindestens 30 erhalten wird.

8. Verfahren nach einem der Ansprüche 1-7, **dadurch gekennzeichnet, dass** die Isomerisationsreaktion und die mindestens eine Extraktion fortgesetzt werden, bis ein Extrakt erhalten wird, welcher 6-Prenylnaringenin und 8-Prenylnaringenin in einem Verhältnis (8-Prenylnaringenin x 100 %)/(8-Prenylnaringenin + 6-Prenylnaringenin) von mindestens 50 %, vorzugsweise mindestens 60 %, bevorzugter mindestens 75 % enthält.

9. Verfahren nach einem der Ansprüche 1-8, **dadurch gekennzeichnet, dass** als ein Hopfenprodukt ein Hopfenprodukt verwendet wird, welches einem zusätzlichen Extraktionsschritt mit Wasser und/oder mindestens einem nichtpolaren organischen Lösemittel unterzogen wurde, gefolgt von der Wiedergewinnung des Restes, welcher den Extrakt angereichert mit prenylierten Flavonoiden enthält.

10. Verfahren nach einem der Ansprüche 1-9, **dadurch gekennzeichnet, dass** die mindestens eine Extraktion mit mindestens einem organischen Lösemittel ausgewählt aus der Gruppe der Alkohole, wasserbasierten Alkohole, Ketone, wasserbasierten Ketone oder Ester oder Mischungen daraus oder basischem Wasser erfolgt.

11. Verfahren nach einem der Ansprüche 1-10, **dadurch gekennzeichnet, dass** die Isomerisationsreaktion bei einer Temperatur zwischen dem Gefrierpunkt und der Siedetemperatur der Reaktionsmischung, vorzugsweise bei Umgebungstemperatur erfolgt.

12. Verfahren nach Anspruch 11, **dadurch gekennzeichnet, dass** die Isomerisationsreaktion bei einer Temperatur zwischen der Umgebungstemperatur und 60 °C, vorzugsweise bei einer Temperatur von etwa der Umgebungstemperatur erfolgt.

13. Verfahren nach einem der Ansprüche 1-12, **dadurch gekennzeichnet, dass** die Isomerisationsreaktion in einer Schutzgasatmosphäre erfolgt.

14. Verfahren nach einem der Ansprüche 1-13, **dadurch gekennzeichnet, dass** die Isomerisationsreaktion für einen Zeitraum zwischen 0,25 und 4 h erfolgt.

15. Verfahren nach einem der Ansprüche 1-14, **dadurch gekennzeichnet, dass** das Verfahren ferner den Schritt des Mischens einer Menge des angereicherten Hopfenextraktes erhalten aus der mindestens einen Extraktion und der Isomerisationsreaktion mit einer Menge eines Hopfenextraktes angereichert mit einem 8-Alkylnaringenin, vorzugsweise 8-Isopentylnaringenin umfasst.

16. Verfahren nach Anspruch 15, **dadurch gekennzeichnet, dass** der Hopfenextrakt angereichert mit 8-Isopentylnaringenin in einem Verfahren erhalten wird, welches folgende Schritte umfasst:
(a) das Unterziehen eines Hopfenextraktes angereichert mit Xanthohumol einer Isomerisationsreaktion zum Umwandeln von Xanthohumol in Isoxanthohumol;
(b) das Unterziehen des Extraktes erhalten in Schritt (a) einer katalytischen Hydrierungsreaktion zum Umwandeln von Isoxanthohumol in Dihydro-Isoxanthohumol;
(c) das Unterziehen des Extraktes erhalten in Schritt (b) einer Demethylierungsreaktion zum Umwandeln von Dihydro-Isoxanthohumol in 8-Isopentylnaringenin.

17. Verfahren nach Anspruch 16, **dadurch gekennzeichnet, dass** die Isomerisationsreaktion in Schritt (a) unter basischen Bedingungen erfolgt.

18. Verfahren nach Anspruch 15-17, **dadurch gekennzeichnet, dass** der Hopfenextrakt angereichert mit 8-Alkylnaringenin durch Zugabe einer Menge eines synthetischen 8-Alkylnaringenin, vorzugsweise einer Menge von synthetischem 8-Isopentylnaringenin erhalten wird.

19. Hopfenextrakt, umfassend eine Mischung aus 8-Prenylnaringenin und 6-Prenylnaringenin, wobei das Verhältnis von (8-Prenylnaringenin x 100 %)/(8-Prenylnaringenin + 6-Prenylnaringenin) mindestens 50 %, vorzugsweise mindestens 60 %, bevorzugter mindestens 75 % beträgt.

20. Hopfenextrakt nach Anspruch 19, **dadurch gekennzeichnet, dass** der Extrakt eine Mischung aus Xanthohumol und 8-Prenylnaringenin umfasst, wobei das Gewichtsverhältnis von Xanthohumol zu 8-Prenylnaringenin mindestens 10, vorzugsweise mindestens 20, bevorzugter mindestens 30 beträgt.

21. Hopfenextrakt nach Anspruch 19 oder 20, **dadurch gekennzeichnet, dass** der Hopfenextrakt mindestens 0,15 % (Gew./Gew.), vorzugsweise mindestens 0,33 % 8-Prenylnaringenin und mindestens 3 %, vorzugsweise mindestens 10 % (Gew./Gew.) Xanthohumol umfasst.

22. Hopfenextrakt nach einem der Ansprüche 19-21, **dadurch gekennzeichnet, dass** der Hopfenextrakt ferner Isoxanthohumol umfasst.

23. Hopfenextrakt nach einem der Ansprüche 19-22, **dadurch gekennzeichnet, dass** der Hopfenextrakt ferner eine Menge 8-Alkylnaringenin, vorzugsweise 8-Isopentylnaringenin umfasst.

24. Verwendung des Hopfenextraktes nach einem der Ansprüche 19-23 oder des Hopfenextraktes erhältlich mit dem Verfahren nach einem der Ansprüche 1-18 für die Herstellung eines Medikamentes oder eines Pflanzenschutzmittels, wobei die mögliche proliferative Aktivität aufgrund der östrogenen Aktivität von 8-Prenylnaringenin durch die antiproliferative Aktivität von Xanthohumol gehemmt (oder ihr entgegengewirkt) wird.

25. Verwendung des Hopfenextraktes nach einem der Ansprüche 19-23 oder des Hopfenextraktes erhältlich mit dem Verfahren nach einem der Ansprüche 1-18 für die Herstellung eines Medikamentes oder eines Pflanzenschutzmittels für die Behandlung oder Prophylaxe von entweder Erkrankungen, Symptomen, Beschwerden oder Krankheitszuständen oder einer Kombination daraus verursacht durch eine Störung im Hormongleichgewicht östrogener Natur.

26. Verwendung nach Anspruch 25, wobei die Erkrankung, das Symptom, die Beschwerden oder der Krankheitszustand verursacht durch die Störung im Hormongleichgewicht östrogener Natur die Menopause ist.

27. Verwendung des Hopfenextraktes nach Anspruch 25, wobei der Krankheitszustand Osteoporose ist.

28. Verwendung des Hopfenextraktes nach Anspruch 25, wobei der Krankheitszustand aus der Gruppe bestehend aus Geschlechtshormonabhängigen Krebsen, kardiovaskulären Erkrankungen, Prostatastörung und Kolonkrebs ausgewählt ist.

29. Nahrungszusammensetzung/-ergänzung, umfassend eine Menge des Hopfenextraktes nach einem der Ansprüche 19-23.

30. Kosmetische Zusammensetzung, umfassend eine Menge des Hopfenextraktes nach einem der Ansprüche 19-23.

## Revendications

1. Procédé pour la production d'un extrait de houblon qui est enrichi en 8-prénylnaringénine par rapport à la 6-prénylnaringénine, **caractérisé en ce que** le procédé comprend les étapes consistant à soumettre des houblons ou un produit à base de houblon (1) à une réaction d'isomérisation en présence d'eau comme solvant et en présence d'une quantité d'une base et (2) à au moins une extraction.

2. Procédé selon la revendication 1, **caractérisé en ce que** la réaction d'isomérisation est effectuée dans de l'eau comme solvant.

3. Procédé selon la revendication 1 ou 2, **caractérisé en ce que** la réaction d'isomérisation est effectuée dans des conditions alcalines correspondant à des concentrations de KOH (% P/V) d'au moins 0,1, de préférence d'au moins 0,5, plus préférablement d'au moins 1, le plus préférablement d'au moins 5.

4. Procédé selon l'une quelconque des revendications 1 - 3, **caractérisé en ce qu'**avant de soumettre des houblons et un produit à base de houblon au ladite au moins une extraction et à la réaction d'isomérisation, les houblons ou le produit à base de houblon est soumis à une extraction en présence de CO₂ liquide ou supercritique ou d'au moins un solvant organique essentiellement non polaire, suivie par la récupération du résidu contenant l'extrait enrichi en flavonoïdes prénylés.

5. Procédé selon l'une quelconque des revendications 1 - 4, **caractérisé en ce que** le procédé comprend en outre le mélange d'une quantité de l'extrait de houblon obtenu par ladite au moins une extraction et la réaction d'isomérisation avec une quantité d'un extrait de houblon enrichi en xanthohumol.

6. Procédé selon l'une quelconque des revendications 1 - 5, **caractérisé en ce que** la réaction d'isomérisation et ladite au moins une extraction sont poursuivies jusqu'à ce que soit obtenu un extrait qui contient au moins 0,15 % en poids, de préférence au moins 0,33 % en poids de 8-prénylnaringénine et au moins 3 % en poids, de préférence au moins 10 % en poids de xanthohumol.

7. Procédé selon l'une quelconque des revendications 1 - 6, **caractérisé en ce que** la réaction d'isomérisation et ladite au moins une extraction sont poursuivies jusqu'à ce que soit obtenu un extrait avec un rapport xanthohumol / 8-prénylnaringénine d'au moins 10, de préférence d'au moins 30.

8. Procédé selon l'une quelconque des revendications 1 - 7, **caractérisé en ce que** la réaction d'isomérisation et ladite au moins une extraction sont poursuivies jusqu'à ce que soit obtenu un extrait qui contient de la 6-prénylnaringénine et de la 8-prénylnaringénine dans un rapport (8-prénylnaringénine x 100 %) / (8-prénylnaringénine + 6-prénylnaringénine) d'au moins 50 %, de préférence d'au moins 60 %, plus préférablement d'au moins 75 %.

9. Procédé selon l'une quelconque des revendications 1 - 8, **caractérisé en ce qu'**on utilise comme produit à base de houblon un produit à base de houblon qui a été soumis à une étape supplémentaire d'extraction avec de l'eau et / ou au moins un solvant organique non polaire, suivie par la récupération du résidu contenant l'extrait enrichi en flavonoïdes prénylés.

10. Procédé selon l'une quelconque des revendications 1 - 9, **caractérisé en ce que** ladite au moins une extraction est effectuée avec au moins un solvant organique choisi dans le groupe des alcools, des alcools à base aqueuse, des cétones, des cétones ou des esters à base aqueuse ou des mélanges de ceux-ci ou de l'eau alcaline.

11. Procédé selon l'une quelconque des revendications 1 - 10, **caractérisé en ce que** la réaction d'isomérisation est effectuée à une température entre le point de congélation et la température d'ébullition du mélange de réaction, de préférence à température ambiante.

12. Procédé selon la revendication 11, **caractérisé en ce que** la réaction d'isomérisation est effectuée à une température entre la température ambiante et 60° C, de préférence à une température proche de la température ambiante.

13. Procédé selon l'une quelconque des revendications 1 - 12, **caractérisé en ce que** la réaction d'isomérisation est effectuée en atmosphère inerte.

14. Procédé selon l'une quelconque des revendications 1 - 13, **caractérisé en ce que** la réaction d'isomérisation est effectuée pendant une période de temps entre 0,25 et 4 h.

15. Procédé selon l'une quelconque des revendications 1 - 14, **caractérisé en ce que** le procédé comprend en outre l'étape consistant à mélanger une quantité de l'extrait de houblon enrichi obtenu à partir de ladite une extraction et de la réaction d'isomérisation avec une quantité d'un extrait de houblon enrichi en une 8-alkylnaringénine, de préférence en 8-isopentylnaringénine.

16. Procédé selon la revendication 15, **caractérisé en ce que** l'extrait de houblon enrichi en 8-isopentylnaringénine est obtenu avec un procédé comprenant les étapes consistant à:
a) soumettre un extrait de houblon enrichi en xanthohumol à une réaction d'isomérisation pour convertir le xanthohumol en isoxanthohumol,
b) soumettre l'extrait obtenu à l'étape (a) à une réaction d'hydrogénation catalytique pour convertir l'isoxanthohumol en dihydro-isoxanthohumol,
c) soumettre l'extrait obtenu à l'étape (b) à une réaction de déméthylation pour convertir le dihydro-isoxanthohumol en 8-isopentylnaringénine.

17. Procédé selon la revendication 16, **caractérisé en ce que** la réaction d'isomérisation à l'étape (a) est effectuée dans des conditions alcalines.

18. Procédé selon les revendications 15 - 17, **caractérisé en ce que** l'extrait de houblon enrichi en 8-alkylnaringénine est obtenu par addition d'une quantité d'une 8-alkylnaringénine synthétique, de préférence d'une quantité de 8-isopentylnaringénine synthétique.

19. Extrait de houblon comprenant un mélange de 8-prénylnaringénine et de 6-prénylnaringénine, dans lequel le rapport de (8-prénylnaringénine x 100 %) / (8-prénylnaringénine + 6-prénylnaringénine) est d'au moins 50 %, de préférence d'au moins 60 %, plus préférablement d'au moins 75 %.

20. Extrait de houblon tel que revendiqué dans la revendication 19, **caractérisé en ce que** l'extrait comprend un mélange de xanthohumol et de 8-prénylnaringénine, le rapport de poids du xanthohumol à la 8-prénylnaringénine étant d'au moins 10, de préférence d'au moins 20, plus préférablement d'au moins 30.

21. Extrait de houblon tel que revendiqué dans la revendication 19 ou 20, **caractérisé en ce que** l'extrait de houblon comprend au moins 0,15 % (poids / poids), de préférence au moins 0,33 % de 8-prénylnaringénine et au moins 3 %, de préférence au moins 10 % (poids / poids) de xanthohumol.

22. Extrait de houblon tel que revendiqué dans l'une quelconque des revendications 19 - 21, **caractérisé en ce que** l'extrait de houblon comprend en outre de l'isoxanthohumol;

23. Extrait de houblon tel que revendiqué dans l'une quelconque des revendications 19 - 22, **caractérisé en ce que** l'extrait de houblon comprend en outre une quantité de 8-alkylnaringénine, de préférence de la 8-isopentylnaringénine.

24. Utilisation de l'extrait de houblon selon l'une quelconque des revendications 19 - 23 ou de l'extrait de houblon pouvant être obtenu avec le procédé de l'une quelconque des revendications 1 - 18 pour la fabrication d'un médicament ou un produit phytopharmaceutique dans lequel l'activité de prolifération possible, due à l'activité oestrogénique de la 8-prénylnaringénine est entravée (ou contrecarrée) par l'activité anti-prolifération du xanthohumol.

25. Utilisation de l'extrait de houblon selon l'une quelconque des revendications 19 - 23 ou de l'extrait de houblon pouvant être obtenu avec le procédé de l'une quelconque des revendications 1 - 18 pour la fabrication d'un médicament ou un produit phytopharmaceutique pour le traitement ou la prophylaxie d'un quelconque des conditions, symptômes, plaintes ou états maladifs ou combinaisons de ceux-ci dus à une perturbation de l'équilibre hormonal de nature oestrogène.

26. Utilisation telle que revendiquée dans la revendication 25, sachant que la condition, le symptôme, la plainte ou l'état maladif dû à la perturbation de l'équilibre hormonal de nature oestrogène est la ménopause.

27. Utilisation de l'extrait de houblon telle que revendiquée dans la revendication 25, sachant que l'état maladif est l'ostéoporose.

28. Utilisation de l'extrait de houblon telle que revendiquée dans la revendication 25, sachant que l'état maladif est choisi dans le groupe consistant en cancers dépendant des hormones sexuelles, en maladies cardiovasculaires, en un dysfonctionnement de la prostate, en un cancer du colon.

29. Composition / complément nutritif comprenant une quantité de l'extrait de houblon selon l'une quelconque des revendications 19 - 23.

30. Composition cosmétique comprenant une quantité de l'extrait de houblon selon l'une quelconque des revendications 19 - 23.
